Europäisches Patentamt

(19) European Patent Office

Office européen des brevets

(11) Numéro de publication : **0 232 202 B1**

(12) **FASCICULE DE BREVET EUROPEEN**

(45) Date de publication du fascicule du brevet :
18.09.91 Bulletin 91/38

(51) Int. Cl.⁵ : **C07H 7/027, C07C 51/31,**
**C08B 31/18, C08B 15/02**

(21) Numéro de dépôt : **87400201.7**

(22) Date de dépôt : **29.01.87**

(54) **Procédé d'oxydation de di-, tri-, oligo- et polysaccharides en acides polyhydroxycarboxyliques, catalyseur mis en oeuvre et produits ainsi obtenus.**

(30) Priorité : 30.01.86 FR 8601305

(43) Date de publication de la demande :
12.08.87 Bulletin 87/33

(45) Mention de la délivrance du brevet :
18.09.91 Bulletin 91/38

(84) Etats contractants désignés :
**AT BE DE ES GB IT NL SE**

(56) Documents cités :
EP-A- 0 142 725
EP-A- 0 151 498
GB-A- 2 075 502
**CHEMICAL ABSTRACTS, volume 103, 1985, page 638, résumé no. 88175q, Columbus, Ohio, US; & JP-A-60 92 240 (KAWAKEN FINE CHEMI-CALS CO. LTD) 23-05-1985**

(56) Documents cités :
**CHEMICAL ABSTRACTS, volume 102, 1985, résumé no. 149721t, Columbus, Ohio, US; & JP-A-59 205 343 (KAO CORP., KAWAKEN FINE CHEMICALS CORP. LTD) 20-11-1984**
**CHEMICAL ABSTRACTS, volume 83, 1975, page 539, résumé no. 43639w, Columbus, Ohio, US; G.L. AKIM et al.: "Radical mecha-nism for the oxidation of carbohydrates with oxygen in an alkaline medium", & TR. VSES. NAUCHNO-ISSLED. INST. TSELLYUL.-BUM PROM-STI. 1974, 64, 190-3**

(73) Titulaire : **Roquette Frères**
**F-62136 Lestrem (FR)**

(72) Inventeur : **Fuertes, Patrick**
**207, rue de Paris**
**F-59000 Lille (FR)**
Inventeur : **Fleche, Guy**
**49, rue Charlet "Le Sart"**
**F-59660 Merville (FR)**

(74) Mandataire : **Koch, Gustave et al**
**Cabinet PLASSERAUD 84, rue d'Amsterdam**
**F-75009 Paris (FR)**

## Description

La présente invention a essentiellement pour objet un procédé d'oxydation de di-, tri-, oligo- et polysaccharides en acides polyhydroxycarboxyliques.

Il est connu de préparer les acides polyhydroxycarboxyliques en oxydant les polysaccharides correspondants par mise en oeuvre de techniques électrochimiques en présence de $CaBr_2$ ; il a également été proposé d'oxyder les fonctions aldéhydes libres des polysaccharides par l'hypochlorite ou l'hypobromite de sodium ; on a enfin proposé l'oxydation catalytique desdits polysaccharides.

Les susdites techniques électrochimiques, outre leur complexité, présentent de nombreux inconvénients, notamment en ce qui concerne la purification des produits obtenus et l'élimination du brome après la réaction d'oxydation. Ils ne sont donc pas économiquement rentables pour une exploitation industrielle.

Les techniques d'oxydation des fonctions aldéhydes libres au moyen de l'hypochlorite ou de l'hypobromite de Na ne sont pas sélectives et entraînent de fait la rupture des liaisons glucosidiques avec génération de produits oxydés de faible masse moléculaire.

L'oxydation catalytique avec recours à des catalyseurs à base de palladium ou platine fixés sur charbon a été décrite dans le brevet français N° 70 18091. Ces procédés manquent de sélectivité et conduisent non seulement à des taux de conversion faibles mais, de plus, ne permettent pas d'éviter des phénomènes de suroxydation, notamment dans le cas du platine. Des perfectionnements ont cependant été décrits dans la demande de brevet EP-A-0142725, relative à la préparation d'acide gluconique par oxydation de glucose sur des catalyseurs préparés par adsorption de bismuth sur du charbon puis adsorption de palladium et réduction subséquante.

L'invention a par conséquence pour but, surtout, de remédier à ces inconvénients et de fournir un procédé d'oxydation sélective des polysaccharides répondant mieux que ceux qui existent déjà aux divers desiderata de la pratique.

Or, la Société Demanderesse a eu le mérite d'établir que, de façon surprenante et inattendue, il était possible, en milieu alcalin, d'oxyder sélectivement et dans des conditions économiques avantageuses les fonctions aldéhydes terminales des polysaccharides en fonction carboxyliques, sans dégradation de la structure polymérique initiale et sans qu'aucune réaction secondaire liée à la rupture des liaisons glucosidiques n'apparaisse, en ayant recours à des catalyseurs à base de métaux nobles du groupe constitué par le palladium, le platine, le rhodium et l'osmium fixés sur support inerte et "dopés" à l'aide d'un ou plusieurs des métaux des groupes IV, V ou VI de la classification périodique qui constituent des "promoteurs".

Il s'ensuit que le procédé de préparation d'acides polyhydroxycarboxyliques conforme à l'invention est essentiellement caractérisé par le fait que l'on oxyde un ou plusieurs polysaccharides en milieu alcalin et au moyen d'un gaz contenant de l'oxygène, en présence d'un catalyseur à base d'un métal noble du groupe constitué par le palladium, le platine, le rhodium et l'osmium fixés sur support inerte et "dopés" à l'aide d'un ou plusieurs des métaux des groupes IV, V ou VI de la classification périodique.

Outre ce procédé de préparation des acides polyhydroxycarboxyliques, l'invention vise les susdits catalyseurs dans le cadre de leur application audit procédé ainsi que les acides polyhydroxycarboxyliques obtenus par la mise en oeuvre de celui-ci.

Les polysaccharides dont le procédé conforme à l'invention rend possible l'oxydation sélective, comprennent :

– les disaccharides possédant une fonction réductrice de type aldose tels que le lactose, le maltose, l'isomaltose, le cellobiose, le xylobiose et le mannobiose, ainsi que

– les trisaccharides, les oligosaccharides et les produits d'hydrolyse de l'amidon, de la cellulose et des hémicelluloses comportant une fonction terminale réductrice de type aldose et le mélange de ces polysaccharides.

L'hydrolyse de l'amidon est généralement effectuée par voie acide et/ou enzymatique et conduit à l'obtention de sirops de glucose. Parmi les produits d'hydrolyse des hémicelluloses, on peut citer les D-galacto-D-mannanes, les D-gluco-D-mannanes, les L-arabino-D-xylanes, les D-xylo-L-arabinanes.

La masse moléculaire des polysaccharides ne constitue pas une limitation à l'invention, dans la mesure où les produits à oxyder sont solubles dans l'eau. Toutefois, les polysaccharides de forte masse moléculaire présentent des viscosités élevées en solution aqueuse, de telle sorte qu'il devient nécessaire d'opérer à faible concentration, ce qui n'est pas avantageux du point de vue industriel.

Dans le cas de l'application du procédé conforme à l'invention à l'oxydation des produits d'hydrolyse de l'amidon, on définit le mélange de polysaccharides résultant de l'hydrolyse par leur pouvoir réducteur ou DE (Dextrose-Equivalent) et par la répartition ou spectre glucidique, le procédé selon l'invention pouvant être mis en oeuvre sur tout hydrolysat d'amidon ou sirop de glucose dont le DE est compris entre 90 et 5 et, de préférence, entre 85 et 15 et, plus préférentiellement encore, entre 75 et 15.

La limite inférieure du DE est imposée, d'une part, par les problèmes de viscosité et de solubilité dont il a été question ci-dessus et, d'autre part, par la cinétique rapidement décroissante avec le degré de polymérisation ou DP.

Les catalyseurs à base de métal noble, notamment à base de palladium et/ou de platine, sont en eux-mêmes connus ; le "support" est généralement constitué par du charbon finement divisé, de l'alumine, de la silice, de la silice-alumine, du sulfate de baryum ou de l'oxyde de titane, le charbon d'une part et le Pd et le Pt d'autre part étant préférés.

La Société Demanderesse a constaté que, de façon surprenante, la présence dans ces catalyseurs d'un ou plusieurs des susdits promoteurs permet d'accroître de manière décisive la cinétique, le rendement et la sélectivité des réactions d'oxydation en milieu alcalin des polysaccharides en acides polyhydroxycarboxyliques.

L'incorporation au catalyseur des susdits promoteurs peut être effectuée avant ou après le dépôt du métal noble sur le support inerte ou de façon simultanée à ce dépôt.

Il est également possible d'introduire le promoteur en solution dans un milieu réactionnel contenant les polysaccharides en solution aqueuse ainsi qu'un catalyseur à base de métal noble. Dans ce cas, le dépôt du promoteur est réalisé in situ dans le milieu réactionnel.

De préférence, les promoteurs sont mis en oeuvre sous la forme de sels pour faciliter leur solubilisation en milieu aqueux, généralement acide.

Pour la préparation du catalyseur "dopé", on mélange la solution de sels de promoteur à une suspension aqueuse du catalyseur constitué par le métal noble, l'imprégnation dudit catalyseur par le promoteur sous forme de sel étant obtenue en maintenant sous agitation le mélange pendant une durée d'au moins quelques secondes à quelques heures, généralement comprise entre 15 minutes et 2 heures.

La suspension ainsi imprégnée de catalyseur supporté à base de métal noble est ensuite rendue alcaline par ajout d'une base telle que la soude, la potasse, le carbonate de sodium et autre, avant que soit réalisée l'étape de réduction du promoteur qui peut être effectuée à une température comprise entre 20°C et 100°C à l'aide d'agents chimiques réducteurs du type formol, formiate de sodium, borohydrure de sodium, acide hypophosphoreux, hydrazine, glucose ou autres sucres réducteurs.

Le catalyseur ainsi réduit est filtré, lavé, séché ou employé tel quel.

On signale que la réduction du catalyseur peut être réalisée au sein du milieu réactionnel d'oxydation catalytique du fait que celle-ci se déroule initialement en présence de polysaccharides réducteurs et en milieu alcalin.

De préférence, on a recours à un catalyseur obtenu par l'addition du promoteur subséquente au dépôt du métal noble sur le support inerte ; on peut ainsi incorporer le promoteur à un catalyseur du commerce à base de métaux nobles sur support inerte.

La teneur en promoteurs du catalyseur final, exprimée en tant que métal, est généralement comprise entre 1 et 300 % en poids par rapport au métal noble.

De préférence toujours, on a recours en tant que promoteur au bismuth, au plomb, à l'antimoine, à l'étain ou au sélénium avec une préférence toute particulière pour le bismuth et le plomb.

Il s'ensuit que les catalyseurs préférés dans le cadre du procédé conforme à la présente invention sont ceux obtenus par dépôt de bismuth et/ou de plomb sur un catalyseur à base de palladium et/ou de platine supporté sur charbon.

La teneur en palladium et/ou en platine exprimée en tant que métal est généralement comprise entre 1 et 10 % en poids par rapport au support.

La teneur en bismuth et/ou en plomb exprimée en tant que métal est comprise entre 1 et 300 % en poids par rapport au palladium et/ou au platine, de préférence entre 5 et 100 %.

Ceci étant, le procédé conforme à l'invention pour la préparation d'acides polyhydroxycarboxyliques et de leurs sels comporte :

– l'introduction d'une solution aqueuse d'au moins un polysaccharide dans une enceinte réactionnelle munie d'un dispositif d'agitation, ladite solution ayant une concentration en polysaccharides comprise de préférence entre 5 et 60 % en poids, la limite inférieure étant dictée par un souci de rentabilité et la limite supérieure par la solubilité de l'oxygène dans les milieux très visqueux, et le risque de cristallisation des sels des acides aldoniques formés pendant la réaction, l'oxydation d'un sirop de glucose étant, à titre d'exemple, effectuée de préférence à une concentration comprise entre 20 et 40 % en poids,

– la dispersion dans cette solution du catalyseur mis en oeuvre conformément à l'invention, la quantité de catalyseur introduite étant telle que la quantité de palladium et/ou de platine exprimée en tant que métal soit comprise entre 0,005 et 1 % en poids par rapport aux polysaccharides et, de préférence, entre 0,01 et 0,4 %,

– l'amorce de la réaction par l'apport simultané d'un balayage d'air ou d'un gaz contenant de l'oxygène et d'un agent alcalin, la température de réaction se situant généralement entre 20 et 90°C, de préférence entre 25 et 60°C pour un temps de réaction compris entre 30 minutes et 5 heures,

– l'introduction dans le milieu réactionnel d'agents alcalins destinée à neutraliser les acides polyhydroxycarboxyliques formés pour maintenir une activité catalytique constante au cours de la réaction et, plus précisément, pour maintenir le pH du milieu réactionnel à une valeur suffisante pour assurer la désorption des acides polyhydroxycarboxyliques formés et éviter la suroxydation de ceux-ci sans que pour autant ce pH atteigne des valeurs susceptibles de favoriser des réactions d'isomérisation d'aldose en cétose, ledit pH étant maintenu dans la pratique à une valeur comprise entre 7,5 et 11,0, de préférence entre 8,0 et 10,0.

L'agent alcalin est choisi dans le groupe comprenant l'hydroxyde de sodium, l'hydroxyde de potassium, l'hydroxyde de calcium, l'hydroxyde de lithium, l'hydroxyde de magnésium en fonction du but visé ; ainsi, on utilisera de la soude pour obtenir le sel de sodium de l'acide polyhydroxycarboxylique correspondant au polysaccharide mis en oeuvre ; on peut également utiliser du carbonate de zinc ou de manganèse ou tous autres sels de zinc ou de manganèse pour lesquels les hydroxydes correspondants sont obtenus in situ par addition d'un agent alcalin tel que l'hydroxyde de sodium ou l'hydroxyde de potassium.

Les catalyseurs mis en oeuvre conformément à l'invention et obtenus par dépôt d'un promoteur, en particulier les catalyseurs palladium-bismuth ou palladium-plomb supportés sur charbon, ont une activité catalytique pratiquement indépendante du degré de polymérisation des polysaccharides soumis à l'oxydation, de telle sorte que la vitesse de réaction reste pratiquement constante lorsque l'on oxyde des mélanges de polysaccharides tels que les produits d'hydrolyse de l'amidon ; on n'observe, dans ce cas, aucune réaction de suroxydation ou de dégradation des polysaccharides.

Le taux de conversion des polysaccharides soumis au procédé conforme à l'invention est supérieur à 90 % et plus particulièrement compris entre 95 et 100 %

Ces performances remarquables sont d'autant plus exceptionnelles qu'elles sont conservées même dans le cas d'un nombre important de recyclage des catalyseurs mis en oeuvre conformément à l'invention.

Les catalyseurs mis en oeuvre préférentiellement, c'est-à-dire ceux dans lesquels le promoteur est déposé après dépôt du métal noble sur le support sont simples à préparer et présentent une grande stabilité et peuvent subir une régénération par dépôt d'une nouvelle charge en promoteur, la quantité de promoteur nécessaire étant, en tout état de cause, toujours faible puisque le dépôt se fait exclusivement à la surface du métal noble.

Les polysaccharides oxydés, obtenus par mise en oeuvre du procédé conforme à l'invention peuvent être utilisés dans de nombreux domaines, notamment sous forme de sel de sodium, à titre d'agents chélatants ou complexants, pour le nettoyage d'articles en verre ou en métal, notamment le fer ou l'aluminium, à titre d'additifs pour détergents, ou dans le domaine des liants hydrauliques en tant que fluidifiants-réducteurs d'eau, retardateurs de prise et autres.

Ils peuvent aussi être utilisés dans le domaine pharmaceutique, comme par exemple le lactobionate de calcium.

L'invention pourra être mieux comprise à l'aide des exemples non limitatifs qui suivent et qui décrivent entre autres des modes de réalisation avantageux de l'invention.

On indique tout d'abord quelques exemples de préparation du catalyseur mis en oeuvre dans le procédé conforme à l'invention.

## EXEMPLE 1

Préparation d'un catalyseur à 5 % de Pd et 3,5 % de Bi sur charbon par dépôt de bismuth sur un catalyseur palladium sur charbon ou Pd/C du commerce.

Une quantité de 6 g de catalyseur sec Pd/C du commerce (DEGUSSA 198 R/W à 5 % Pd) est mise en suspension dans 80 ml d'eau distillée acidifiée par 1 ml d'acide chlorhydrique concentré (37 % HCl). A cette suspension, on ajoute une solution constituée de 0,3 g de subnitrate de bismuth dissous dans un mélange de 2 ml d'acide chlorhydrique concentré et de 5 ml d'eau distillée.

On établit une agitation pendant deux heures, puis on introduit 4 g de soude caustique en solution dans 30 ml d'eau. Le mélange est porté à une température de 40-50°C pendant 4 heures, puis on ajoute 1,5 ml de formol (solution aqueuse à 37-38%). On porte le mélange à 85°C pendant 1 heure. Le catalyseur ainsi obtenu est filtré et lavé.

## EXEMPLE 2

Préparation d'un catalyseur à 5 % de Pd et 3,5 % de Bi sur charbon par dépôt de bismuth avant le dépôt de palladium.

Une quantité de 6 g de charbon actif sec est mise en suspension dans 80 ml d'eau distillée. A cette suspension, on ajoute une solution constituée de 0,3 g de subnitrate de bismuth dissous dans un mélange de 3 ml d'acide chlorhydrique concentré et de 5 ml d'eau distillée.

On établit une agitation pendant 6 heures afin de laisser le bismuth s'adsorber complètement sur le charbon actif. On ajoute ensuite une solution de 0,5 g de chlorure de palladium (0,3 g de palladium métal) dans 1,5 ml d'acide chlorhydrique et 5 ml d'eau distillée. On ajoute 4 g de soude caustique en solution dans 30 ml d'eau et on porte le mélange à 40°C pendant 5 heures. Après addition de 1,5 ml d'une solution de formol à 37 %, on maintient la suspension à 85°C pendant 1 heure. Le catalyseur ainsi obtenu est filtré et lavé.

## EXEMPLE 3

Préparation d'un catalyseur à 5 % de Pd et 2,5 % de Pb sur charbon par dépôt de plomb sur un catalyseur Pd/C du commerce.

Une quantité de 6 g de catalyseur Pd/C sec du commerce (DEGUSSA 198 R/W à 5 % Pd) est mise en suspension dans 80 ml d'eau distillée. On ajoute à cette suspension 20 ml d'une solution aqueuse contenant 0,3 g d'acétate de plomb. On laisse adsorber le plomb pendant 1 heure sous agitation. On ajoute 30 ml d'une solution aqueuse contenant 4 g de $Na_2CO_3$ et le mélange obtenu est porté à 40°C pendant 4 heures. On ajoute 1,5 ml de formol et on maintient la suspension à 85°C pendant 1 heure. Le catalyseur ainsi obtenu est ensuite filtré et lavé à l'eau distillée.

On indique à présent quelques exemples de mise en oeuvre du procédé conforme à l'invention.

## EXEMPLE 4

Préparation du maltobionate de sodium par oxydation du maltose.

On réalise quatre expériences en introduisant chaque fois individuellement dans une enceinte réactionnelle d'une capacité de 1 litre, équipée d'un agitateur et d'un thermomètre, d'une tige frittée d'insufflation d'air, d'une électrode et d'un dispositif d'introduction en continu, une quantité de 666 g d'une solution aqueuse de maltose à 30 % de matières sèches ainsi qu'une quantité de 6 g de respectivement chacun des catalyseurs secs selon les exemples 1 à 3 pour les trois premières expériences (expériences a, b et c) et une quantité de 6 g du catalyseur sec du commerce utilisé dans les exemples 1 et 3 pour la quatrième expérience (expérience d).

On fait réagir à 35°C et on insuffle de l'air tout en introduisant simultanément une solution aqueuse de soude à 30 % de façon à maintenir le pH à une valeur de 9,0 ±0,5.

On arrête la réaction lorsque la quantité théorique de soude a été consommée, ce qui donne la vitesse de la réaction ; le produit réactionnel est ensuite séparé par filtration et on détermine le pourcentage de sucres réducteurs, ce qui permet de calculer le taux de conversion du substrat.

Le maltose utilisé dans l'expérience c est un maltose à 99,9 % de pureté et celui utilisé dans les expériences, a, b et d est à 95 % de pureté.

Dans le tableau I, on a réuni les résultats des susdites mesures et déterminations.

EP 0 232 202 B1

### TABLEAU I

| No. de l'expérience | Catalyseur utilisé | Temps de réaction | Sucres réducteurs (%) | Taux de conversion du maltose |
|---|---|---|---|---|
| a | 5% Pd et 3,5% Bi sur charbon (exemple 1) | 1 h 40 | 1,3 | 97,5 |
| b | 5% Pd et 3,5% Bi sur charbon (exemple 2) | 1 h 50 | 1,4 | 97,0 |
| c | 5% Pd et 3,5% Bi sur charbon (exemple 1) | 0 h 40 | 0,6 | 98,9 |
| d | 5% Pd/C du commerce (DEGUSSA 198 R/W) | 4 h 40 | 2,6 | 95,1 |

## EXEMPLE 5

Préparation du lactobionate de sodium par oxydation du lactose.

Le mode opératoire suivi pour la réalisation de cet exemple est identique à celui décrit à l'exemple 4 mise à part la quantité de matière première mise en oeuvre qui est de 1000 g de solution à 20 % en poids de lactose d'une pureté de 99,8 %.

Les catalyseurs utilisés sont, d'une part, celui de l'exemple 1 et, d'autre part, un catalyseur Pd/C du commerce (DEGUSSA C 101 R/W à 5 % Pd).

Les données numériques de l'exemple 5 sont réunies dans le tableau II.

### TABLEAU II

| Catalyseur utilisé | Temps de réaction | Sucres réducteurs (%) | Taux de conversion du lactose (%) |
|---|---|---|---|
| 5% Pd et 3,5% Bi sur charbon (exemple 1) | 1 h 20 | 4,6 | 91 |

Dans le cas du catalyseur du commerce et après 4 heures de réaction, la quantité de soude consommée était inférieure de 10 % à la quantité théorique. Dans ces conditions, l'essai n'a pas été poursuivi.

## EXEMPLE 6

Préparation d'acides polyhydroxycarboxyliques par oxydation de polysaccharides.

Une série de 12 expériences est réalisée avec, comme matière première, huit produits d'hydrolyse de l'amidon dont le DE varie entre 90 et 27,7.

Le mode opératoire retenu pour l'oxydation des matières premières identifiées dans le tableau III est en tout point identique à celui indiqué pour l'exemple 4.

Les catalyseurs sont soit ceux selon les exemples 1 à 3, soit le catalyseur du commerce utilisé dans ces exemples 1 et 3.

6

On détermine toujours de la même manière le temps de réaction et, sur le produit fini, le pourcentage de sucres réducteurs résiduels, ce qui a permis de calculer le taux de conversion des polysaccharides.

EP 0 232 202 B1

**TABLEAU III**

| No. de l'expé-rience | Définition de l'hydrolysat | DE de l'hydrolysat | Spectre glucidique | | | | | | | | | | |
|---|---|---|---|---|---|---|---|---|---|---|---|---|---|
| | | | glu-cose | mal-tose | DP3 | DP4 | DP5 | DP6 | DP7 | DP8 | DP9 | DP10-20 | DP20 |
| e | Hydrolysat à haut DE | 90 | 85,4 | 9,4 | 3,9 | 2 | | | | | | | |
| f | Hydrolysat à moyen DE * | 84 | 71,5 | 14,0 | 4,2 | 6,1 | | | | | | | |
| g | Sirop de glucose riche en maltose | 65,8 | 40,2 | 28,3 | 10,2 | 1,6 | 0,8 | 0,8 | 1,2 | 1,7 | 2,4 | 8,7 | 4,0 |
| h | Sirop de glucose à haut DE | 40,8 | 17,1 | 14,6 | 10,7 | 8,1 | 6,5 | 5,0 | 4,5 | 4,5 | 3,3 | 20,6 | 5,0 |
| i | Sirop de glucose à moyen DE | 38 | 14,2 | 11,4 | 9,8 | 8,8 | 7,8 | 5,7 | 5,1 | 4,5 | 3,9 | 23,0 | 5,9 |
| j | Sirop de glucose à bas DE | 33,1 | 11,4 | 9,6 | 8,4 | 7,5 | 6,7 | 5,1 | 4,8 | 4,4 | 4,0 | 27,3 | 10,9 |
| k | Sirop de glucose à faible teneur en polysaccharides | 39,8 | 13,1 | 13 | 13 | 7,7 | 10,8 | 11,5 | 2,8 | 2,7 | 2,7 | 17,5· | 4,8 |
| m | Maltodextrine à moyen DE | 27,7 | 9,3 | 7,7 | 7,7 | 7,8 | 6,6 | 5,9 | 5,1 | 4,7 | 4,2 | 29,2 | 11,2 |

* teneur en fructose 4,1 %

Dans le tableau IV, on a identifié pour les douze expériences la matière première et le catalyseur utilisés et on a indiqué chaque fois le résultat des susdites déterminations.

## TABLEAU IV

| Matière première | DE | Catalyseur | Temps de réaction | Sucres réducteurs (en %) | Taux de conversion des polysac- charides (%) |
|---|---|---|---|---|---|
| Hydrolysat à haut DE (e) | 90,0 | Pd/Bi/C selon l'exemple 1 | 2 h 40 | 1,6 | 97,9 |
| Hydrolysat à moyen DE (f) | 84,0 | Pd/Bi/C selon l'exemple 1 | 2 h 30 | 6,8* | 97,0 |
| Sirop de glucose (g) riche en maltose | 65,8 | Pd/Bi/C selon l'exemple 1 | 4 h 30 | 1,70 | 97,5 |
| Sirop de glucose (g) riche en maltose | 65,8 | Pd/Bi/C selon l'exemple 2 | 4 h 20 | 1,80 | 97,2 |
| Sirop de glucose (g) riche en maltose | 65,8 | Pd/Bi/C selon l'exemple 3 | 4 h 30 | 1,80 | 97,2 |
| Sirop de glucose (g) riche en maltose | 65,8 | Pd/C du commerce | 7 h 30 | 11,40 | 82,7 |
| Sirop de glucose (h) à haut DE | 40,8 | Pd/Bi/C selon l'exemple 1 | 3 h 20 | 1,85 | 95,5 |
| Sirop de glucose (i) à moyen DE | 38,0 | Pd/Bi/C selon l'exemple 1 | 2 h 50 | 1,55 | 96,0 |
| Sirop de glucose (i) à moyen DE | 38,0 | Pd/C du du commerce | 7 h 45 | 15,0 | 60,0 |
| Sirop de glucose (j) à bas DE | 33,1 | Pd/Bi/C selon l'exemple 1 | 3 h 00 | 1,70 | 94,9 |
| Sirop de glucose (j) à bas DE | 40,8 | Pd/Bi/C selon l'exemple 3 | 3 h 05 | 2,50 | 92,5 |
| Maltodextrine (m) à moyen DE | 27,7 | Pd/Bi/C selon l'exemple 1 | 2 h 20 | 2,30 | 92,0 |

* teneur en fructose 4,1 %

L'examen des résultats réunis dans le tableau IV permet de parvenir aux conclusions développées ci-après.

L'oxydation catalytique des polysaccharides à l'aide d'un catalyseur Pd/Bi ou Pd/Pb sur charbon permet d'atteindre un taux de conversion des polysaccharides de 92 à 97 % pour des produits dont le DE et la répartition glucidique varient dans un domaine très large. L'oxydation réalisée avec ces catalyseurs préférentiels est pratiquement totale et n'est pas affectée par la présence de polysaccharides dont le degré de polymérisation est supérieur à 10. La faible teneur en sucres réducteurs, ainsi que l'analyse chromatographique High Performance Liquid Chromatography (HPLC)) indiquent que tous les polysaccharides sont oxydés et que la répartition glucidique est intégralement conservée.

Par contre, les essais effectués avec un catalyseur Pd/C du commerce montrent que le temps de réaction augmente de façon significative, ainsi que la teneur en sucres réducteurs. La différence est d'autant plus marquée que le D.E. initial est faible. Cette observation traduit la difficulté d'oxydation des polysaccharides dont

9

le degré de polymérisation est élevé.

*Plus précisément, dans le cas de l'oxydation catalytique d'un sirop de glucose à moyen DE, avec un catalyseur Pd/C du commerce non dopé, on observe par chromatographie HPLC que seule la fraction glucose est oxydée totalement. La fraction maltose est oxydée à 50% environ, la fraction maltotriose est oxydée à 30% environ et les polysaccharides supérieurs ne subissent qu'une oxydation mineure. Les catalyseurs Pd/C du commerce sont donc relativement inadaptés à l'oxydation totale des polysaccharides.*

Comme il va de soi et comme il résulte d'ailleurs déjà de ce qui précède, l'invention ne se limite nullement à ceux de ses modes d'application et de réalisation qui ont été plus spécialement envisagés ; elle en embrasse, au contraire, toutes les variantes.

## Revendications

1. Procédé d'oxydation sélective de di-, tri-, oligo- et polysaccharides comportant une fonction terminale réductrice du type aldose en acides polyhydroxycarboxyliques, caractérisé par le fait que l'oxydation est réalisée en milieu alcalin, au moyen d'un gaz contenant de l'oxygène, en présence d'un catalyseur à base de métal noble choisi dans le groupe constitué par le palladium, le platine, le rhodium et l'osmium et fixé sur support inerte, ledit catalyseur étant "dopé" à l'aide d'un ou plusieurs métaux, ou promoteurs, des groupes IV, V ou VI de la classification périodique.

2. Procédé selon la revendication 1, caractérisé par le fait que le promoteur est choisi parmi le bismuth, le plomb, l'antimoine et le sélénium.

3. Procédé selon l'une des revendications 1 ou 2, caractérisé par le fait que le support inerte est choisi parmi le charbon, l'alumine, la silice, la silice-alumine, le sulfate de baryum et l'oxyde de titane.

4. Procédé selon l'une des revendications 1 à 3, caractérisé par le fait que l'on a recours, à titre de catalyseur, à ceux obtenus par dépôt de bismuth et/ou de plomb sur un catalyseur à base de palladium et/ou de platine supportés sur charbon.

5. Procédé selon l'une des revendications 1 à 4, caractérisé par le fait que la teneur du catalyseur en palladium et/ou en platine, exprimée en tant que métal, est comprise entre 1 et 10 % en poids par rapport au support.

6. Procédé selon l'une des revendications 1 à 5, caractérisé par le fait que la teneur du catalyseur en bismuth et/ou en plomb, exprimée en tant que métal, est comprise entre 1 et 300 % en poids par rapport au palladium et/ou au platine, de préférence entre 5 et 100 %.

7. Procédé selon l'une des revendications 1 à 6, caractérisé par le fait que les di-, tri-, oligo- et polysaccharides comportant une fonction réductrice de type aldose sont mis en oeuvre sous la forme d'une solution aqueuse d'une concentration comprise entre 5 et 60 % en poids.

8. Procédé selon l'une des revendications 1 à 6, caractérisé par le fait que l'on met en oeuvre un hydrolysat d'amidon ou sirop de glucose d'une concentration comprise de préférence entre 20 et 40 % en poids.

9. Procédé selon l'une des revendications 1 à 8, caractérisé par le fait que les di-, tri-, oligo- et polysaccharides comportant une fonction réductrice de type aldose sont mis en oeuvre sous la forme d'hydrolysats d'amidon ou sirops de glucose dont le DE (dextrose equivalent( est compris entre 90 et 5, de préférence entre 85 et 15 et, plus préférentiellement encore, entre 75 et 15.

10. Procédé selon l'une des revendications 1 à 9, caractérisé par le fait que le catalyseur est dispersé dans la solution de di-, tri-, oligo- et polysaccharides en une quantité telle que la concentration en palladium et/ou en platine, exprimée en tant que métal, soit comprise entre 0,005 et 1 % en poids par rapport aux polysaccharides et, de préférence, entre 0,01 et 0,4 %.

11. Procédé selon l'une des revendications 1 à 10, caractérisé par le fait que la température à laquelle est conduite la réaction d'oxydation est comprise entre 20 et 90°C, de préférence entre 25 et 60°C, pour un temps de réaction compris entre 30 minutes et 5 heures.

12. Procédé selon l'une des revendications 1 à 11, caractérisé par le fait que le pH du milieu réactionnel est maintenu à l'aide d'un ou plusieurs agents alcalins à une valeur comprise entre 7,5 et 11,0, de préférence entre 8,0 et 10,0.

13. Procédé selon l'une des revendications 1 à 12, caractérisé par le fait que l'agent alcalin est choisi dans le groupe comprenant l'hydroxyde de calcium, l'hydroxyde de lithium, l'hydroxyde de magnésium, le carbonate de zinc ou de manganèse, ou tous autres sels de zinc ou de manganèse pour lesquels les hydroxydes correspondants sont obtenus in situ par addition d'un agent alcalin tel que l'hydroxyde de sodium ou l'hydroxyde de potassium.

14. Application à la préparation des acides polyhydroxycarboxyliques par oxydation sélective des fonctions aldéhyde terminales de di-, tri-, oligo- et polysaccharides d'un catalyseur à base d'un métal noble du groupe

EP 0 232 202 B1

constitué par le palladium, le platine, le rhodium et l'osmium fixés sur support inerte, ledit catalyseur étant 'dopé" à l'aide d'un ou plusieurs des métaux ou promoteurs des groupes IV, V ou VI de la classification périodique, ledit promoteur étant de préférence choisi parmi le bismuth, le plomb, l'antimoine, l'étain et le sélénium, le support inerte étant de préférence choisi parmi le charbon finement divisé, l'alumine, la silice, la silice-alumine, le sulfate de baryum et l'oxyde de titane.

15. Application des acides polyhydroxycarboxyliques obtenus par mise en oeuvre du procédé selon l'une des revendications 1 à 14, notamment sous forme de sel de sodium, à titre d'agents chélatants ou complexants, pour le nettoyage d'articles en verre ou en métal, notamment le fer ou l'aluminium, à titre d'additifs pour détergents ou dans le domaine des liants hydrauliques en tant que fluidifiants-réducteurs d'eau, retardateurs de prise et autres.

## Patentansprüche

1. Verfahren zur selektiven Oxidation von Di-, Tri-, Oligo- und Polysacchariden, welche eine endständige, reduzierende Funktion vom Aldosetypus enthalten, zu Polyhydroxycarbonsäuren, dadurch gekennzeichnet, daß die Oxidation in einem alkalischen Medium mit Hilfe eines sauerstoffhältigen Gases und in Gegenwart eines Katalysators auf der Basis eines Edelmetalles durchgeführt wird, welches Edelmetall aus der aus Palladium, Platin, Rhodium und Osmium bestehenden Gruppe ausgewählt und auf einem inerten Träger aufgebracht ist und welcher Katalysator mit Hilfe einer oder mehrerer Metalle oder Promotoren aus den Gruppen IV, V oder VI des Periodensystems "dotiert" ist.

2. Verfahren nach Anspruch 1, dadurch gekennzeichnet, daß der Promotor aus Wismut, Blei, Antimon und Selen ausgewählt wird.

3. Verfahren nach Anspruch 1 oder 2, dadurch gekennzeichnet, daß der inerte Träger aus Kohle, Aluminiumoxid, Kieselsäureanhydrid, Kieselsäureanhydrid-Aluminiumoxid, Bariumsulfat und Titanoxid ausgewählt ist.

4. Verfahren nach einem der Ansprüche 1 bis 3, dadurch gekennzeichnet, daß man als Katalysator solche verwendet, die durch Aufbringung von Wismut und/oder Blei auf einen Katalysator auf der Basis von Palladium und/oder Platin, das bzw. die auf einem Kohleträger vorliegt bzw. vorliegen, erhalten worden sind.

5. Verfahren nach einem der Ansprüche 1 bis 4, dadurch gekennzeichnet, daß der Gehalt des Katalysators an Palladium und/oder Platin, als Metall ausgedrückt, zwischen 1 und 10 Gew.-%, bezogen auf den Träger, beträgt.

6. Verfahren nach einem der Ansprüche 1 bis 5, dadurch gekennzeichnet, daß der Gehalt des Katalysators an Wismut und/oder Blei, ausgedrückt als Metall, zwischen 1 und 300 Gew.-% bezogen auf das Palladium und-/oder Platin, vorzugsweise zwischen 5 und 100 Gew.-% beträgt.

7. Verfahren nach einem der Ansprüche 1 bis 6, dadurch gekennzeichnet, daß die Di-, Tri-, Oligo- und Polysaccharide, welche eine reduzierende Funktion vom Aldosetypus enthalten, in der Form einer wässerigen Lösung mit einer zwischen 5 und 60 Gew.-% liegenden Konzentration eingesetzt werden.

8. Verfahren nach einem der Ansprüche 1 bis 6, dadurch gekennzeichnet, daß man ein Stärkehydrolysat oder einen Glucosesirup mit einer vorzugsweise zwischen 20 und 40 Gew.-% liegenden Konzentration einsetzt.

9. Verfahren nach einem der Ansprüche 1 bis 8, dadurch gekennzeichnet, daß die Di-, Tri-, Oligo- und Polysaccharide mit einem Gehalt an einer reduzierenden Funktion vom Aldosetypus in der Form von Stärkehydrolysaten oder von Glucosesirupen eingesetzt werden, deren DE (Dextroseäquivalent) zwischen 90 und 5, vorzugsweise zwischen 85 und 15, und in noch höher bevorzugtem Maße zwischen 75 und 15 liegt.

10. Verfahren nach einem der Ansprüche 1 bis 9, dadurch gekennzeichnet, daß der Katalysator in der Lösung der Di-,Tri-,Oligo- und Polysaccharide in einer solchen Menge dispergiert wird, daß die Konzentration an Palladium und/oder Platin, als Metall ausgedrückt, zwischen 0,005 und 1 Gew.-%, vorzugsweise zwischen 0,01 und 0,4 Gew.-%, bezogen auf die Polysaccharide, beträgt.

11. Verfahren nach einem der Ansprüche 1 bis 10, dadurch gekennzeichnet, daß die Temperatur, bei welcher die Oxidationsreaktion durchgeführt wird, zwischen 20 und 90° C, vorzugsweise zwischen 25 und 60° C, liegt, und daß die Reaktionszeit zwischen 30 min und 5 h beträgt.

12. Verfahren nach einem der Ansprüche 1 bis 11, dadurch gekennzeichnet, daß der pH-Wert des Reaktionsmediums mit Hilfe eines oder mehrerer alkalischer Mittel auf einem zwischen 7,5 und 11,0, vorzugsweise zwischen 8,0 und 10,0 liegenden Wert gehalten wird.

13. Verfahren nach einem der Ansprüche 1 bis 12, dadurch gekennzeichnet, daß das alkalische Mittel aus einer Calciumhydroxid, Lithiumhydroxid, Magnesiumhydroxid, die Carbonate von Zink oder Mangan, oder jedwede andere Salze von Zink oder Mangan, für welche die entsprechenden Hydroxide in situ durch Zugabe eines alkalischen Mittels, wie z.B. von Natriumhydroxid oder Kaliumhydroxid, erhalten werden, umfassenden

11

EP 0 232 202 B1

Gruppe ausgewählt.wird.

14. Verwendung eines Katalysators auf der Basis eines Edelmetalles, welches aus der aus Palladium, Platin, Rhodium und Osmium bestehenden Gruppe ausgewählt ist, und welches auf einem inerten Träger aufgebracht ist, wobei dieser Katalysator mit Hilfe von einem oder mehreren Metallen oder Promotoren aus den Gruppen IV, V oder VI des Periodensystems "dotiert" worden ist und wobei dieser Promotor vorzugsweise aus Wismut, Blei, Antimon, Zinn und Selen ausgewählt ist, und wobei der inerte Träger vorzugsweise aus fein verteilter Kohle, Aluminiumoxid, Kieselsäureanhydrid, Kieselsäureanhydrid-Aluminiumoxid, Bariumsulfat und Titanoxid ausgewählt ist, für die Herstellung von Polyhydroxycarbonsäuren durch selektive Oxidation der endständigen Aldehydfunktionen von Di-, Tri-, Oligo- und Polysacchariden.

15. Verwendung von Polyhydroxycarbonsäuren, welche nach Durchführung des Verfahrens nach einem der Ansprüche 1 bis 14 erhalten worden sind, insbesondere in der Form des Natriumsalzes, als Chelatbildner oder als Komplexbildner, als Mittel zum Reinigen von Gegenständen aus Glas bzw. aus Metall, insbesondere Eisen oder Aluminium, als Zusatzstoffe für Detergenzien, oder auf dem Gebiet der hydraulischen Bindemittel als Fluidifizierungsmittel-Wasserverringerungsmittel, Abbindeverzögerer, u. dgl.

## Claims

1. Process for the selective oxidation of di-, tri-, oligo- and polysaccharides comprising a reducing terminal function of the aldose type to polyhydroxycarboxylic acids, characterized by the fact that the said oxidation is carried out in alkaline medium by means of an oxygen-containing gas, in the presence of a catalyst based on a noble metal selected from the group constituted by palladium, platinum, rhodium and osmium and fixed on an inert support, said catalyst being "doped" with one or several metals, or promoters, of Groups IV, V or VI of the Periodic Table.

2. Process according to claim 1, characterized by the fact that the promoter is selected from bismuth, lead, antimony and selenium.

3. Process according to one of claims 1 or 2, characterized by the fact that the inert support is selected from carbon, alumina, silica, silica-alumina, barium sulfate and titanium oxide.

4. Process according to one of claims 1 to 3, characterized by the fact that as catalyst there are used those obtained by depositing bismuth and/or lead onto a palladium and/or platinum based catalyst, supported on carbon.

5. Process according to one of claims 1 to 4, characterized by the fact that the palladium and/or platinum content of the catalyst, expressed in terms of metal, is comprised between 1 and 10 wt% with respect to the support.

6. Process according to one of claims 1 to 5, characterized by the fact that the bismuth and/or lead content of the catalyst, expressed in terms of metal, is comprised between 1 and 300 wt% with respect to palladium and/or platinum, preferably between 5 and 100 %.

7. Process according to one of claims 1 to 6, characterized by the fact that the di-, tri-, oligo- and polysaccharides comprising a reducing function of the aldose type are used in the form of an aqueous solution having a concentration comprised between 5 and 60 wt%.

8. Process according to one of claims 1 to 6, characterized by the fact that a starch hydrolysate or a glucose syrup having a concentration preferably comprised between 20 and 40 wt%, is used.

9. Process according to one of claims 1 to 8, characterized by the fact that the di-, tri-, oligo- and polysaccharides comprising a reducing function of the aldose type are used in the form of starch hydrolysates or glucose syrups whose D.E. (dextrose equivalent) is comprised between 90 and 5, preferably between 85 and 15, and, still more preferably, between 75 and 15.

10. Process according to one of claims 1 to 9, characterized by the fact that the catalyst is dispersed in the solution of di-, tri, oligo- and polysaccharides in an amount such that the palladium and/or platinum concentration, expressed in terms of metal, is comprised between 0.005 and 1 wt% with respect to the polysaccharides and, preferably, between 0.01 and 0.4 wt%.

11. Process according to one of claims 1 to 10, characterized by the fact that the temperature at which the reaction of oxidation is, carried out, is comprised between 20 and 90°C, preferably between 25 and 60°C, the reaction time being comprised between 30 minutes and 5 hours.

12. Process according to one of claims 1 to 11, characterized by the fact that the pH of the reaction medium is maintained by means of one or several alkaline agents at a value comprised between 7.5 and 11.0, preferably between 8.0 and 10.0.

13. Process according to one of claims 1 to 12, characterized by the fact that the alkaline agent is selected from the group comprising calcium hydroxide, lithium hydroxide, magnesium hydroxide, zinc or manganese car-

12

bonate, or any other zinc or manganese salts for which the corresponding hydroxides are obtained in situ by adding an alkaline agent such as sodium hydroxide or potassium hydroxide.

14. Use for the preparation of polyhydroxycarboxylic acids by means of the selective oxidation of the terminal aldehyde functions of di-, tri-, oligo- and polysaccharides of a catalyst based on a noble metal from the group constituted by palladium, platinum, rhodium and osmium, fixed on an inert support, said catalyst being "doped" with one or several of the metals or promoters from Groups IV, V or VI of the Periodic Table, said promoter being preferably selected from bismuth, lead, antimony, tin and selenium, the inert support being preferably selected from finely divided carbon, alumina, silica, silica-alumina, barium sulfate and titanium oxide.

15. Use of the polyhydroxycarboxylic acids obtained by carrying out the process according to one of claims 1 to 14, the said acids being especially in the form of sodium salt, as chelating or complexing agents, for the cleaning of glass or metal articles or items, especially of iron or aluminum, as additives for detergents or in the field of the hydraulic binders as fluidifying agents reducing water, concrete retarding admixtures and other.